(19)

Europäisches
Patentamt
European
Patent Office
Office européen
des brevets

(11) **EP 2 937 140 A1**

(12) **EUROPEAN PATENT APPLICATION**
published in accordance with Art. 153(4) EPC

(43) Date of publication:
28.10.2015 Bulletin 2015/44

(21) Application number: 14800472.4

(22) Date of filing: 26.05.2014

(51) Int Cl.:
*B01J 23/31* $^{(2006.01)}$          *B01J 35/02* $^{(2006.01)}$
*B01J 37/08* $^{(2006.01)}$          *C07C 57/04* $^{(2006.01)}$
*C07C 47/22* $^{(2006.01)}$

(86) International application number:
**PCT/KR2014/004670**

(87) International publication number:
**WO 2014/189341 (27.11.2014 Gazette 2014/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: 24.05.2013 KR 20130059095
23.05.2014 KR 20140062552

(71) Applicant: LG Chem, Ltd.
Seoul 150-721 (KR)

(72) Inventors:
• LIM, Hyun-Sub
Daejeon 305-738 (KR)

• SHIN, Hyun-Jong
Daejeon 305-738 (KR)
• PARK, Ju-Yeon
Daejeon 305-738 (KR)
• CHOI, Byung-Yul
Daejeon 305-738 (KR)
• CHOE, Young-Hyun
Daejeon 305-738 (KR)
• KIM, Duk-Ki
Daejeon 305-738 (KR)

(74) Representative: Goddar, Heinz J.
Boehmert & Boehmert
Anwaltspartnerschaft mbB
Patentanwälte Rechtsanwälte
Pettenkoferstrasse 20-22
80336 München (DE)

(54) **RING CATALYST FOR PREPARING ACROLEIN AND ACRYLIC ACID, AND USE THEREOF**

(57) The present invention relates to a catalyst for preparing acrolein and acrylic acid, and use of the same. The catalyst according to the present invention can be uniformly packed in the reactor and collapse of the catalyst can be minimized because it has excellent mechanical properties, and it can lower initial pressure of the reaction by securing stable pore spaces and can be stably used for a long period of time. In addition, the preparation method of acrolein and acrylic acid according to the present invention can provide more improved production efficiency by using the catalyst.

[Fig. 1]

(a)

(b)

EP 2 937 140 A1

**Description**

**[TECHNICAL FIELD]**

The present invention relates to a ring-shaped catalyst for preparing acrolein and acrylic acid, and use of the same.

**[BACKGROUND OF ART]**

A method of carrying out vapor-phase oxidation of propylene, isobutylene, or tert-butanol and molecular oxygen in a multi-tubular fixed bed reactor including a catalyst layer is generally being used for preparing acrolein and/or acrylic acid.

However, said reaction is an exothermal reaction, and thus various methods of limiting the thickness of the catalyst layer, using a supported catalyst in which a catalytic active material is loaded, or using a catalyst formed into a cylinder shape or a ring shape are being applied in order to minimize the temperature rise during the reaction.

Among them, the catalyst of a ring shape (that is, a catalyst of a hollow cylinder shape, hereinafter 'ring catalyst') can secure wider pore space than pellet catalysts, and thus can exhibit a wider surface area of the catalytic active ingredient and more improved heat dissipation ability.

However, the ring catalyst is easily broken by a shock in the processes of preparation, storage, or packing in the reaction tube because the structure of the same is inferior to that of the pellet catalyst in terms of mechanical properties. Further, there is a problem that the broken fragments or dust of the ring catalyst raise the pressure in the reaction tube and make the pressure of each reaction tube irregular, particularly in the case of a multi-tubular reactor. Furthermore, the ring catalyst has a problem that the life span of the catalyst is limited because less of the catalytic active ingredient is packed in the reaction tube than the pellet catalyst.

**[DETAILED DESCRIPTION OF THE INVENTION]**

**[Technical Problem]**

It is an aspect of the present invention to provide a ring catalyst for preparing acrolein and acrylic acid having more improved mechanical properties than prior ring catalysts.

It is another aspect of the present invention to provide a preparation method of acrolein and acrylic acid using the ring catalyst.

**[Technical Solution]**

According to the present invention,
a ring catalyst for preparing acrolein and acrylic acid,
including a mixture of an catalytic active ingredient including at least molybdenum (Mo) and bismuth (Bi), and an inorganic fiber, and
having a ring shape, and
satisfying the following Relational Equation 1,
is provided.

[Relational Equation 1]

$$0.1 \leq [2L_f/(D_e-D_i)] < 0.2$$

In said Relational Equation 1, $L_f$ is the number average length of the inorganic fiber, $D_e$ is the external diameter of the ring catalyst, and $D_i$ is the internal diameter of the ring catalyst.

The catalyst may satisfy the following Relational Equation 2.

[Relational Equation 2]

$$0.2 \leq L_c/D_e \leq 1.5$$

In said Relational Equation 2, $L_c$ is the longitudinal length of the ring catalyst, and $D_e$ is the external diameter of the ring catalyst.

The catalytic active ingredient may be represented by the following Chemical Formula 1.

[Chemical Formula 1]     $MO_a\ Bi_b\ A_c\ B_d\ C_e\ Of$

In Chemical Formula 1,

Mo is molybdenum; Bi is bismuth; A is one or more elements selected from the group consisting of Fe, Zn, Mn, Nb, and Te; B is one or more elements selected from the group consisting of Co, Rh, and Ni; C is one or more elements selected from the group consisting of Na, K, Li, Cs, Ta, Ca, Rb, and Mg; O is oxygen; and

a, b, c, d, e, and f are atomic ratios of each element, wherein b is 0.1 to 10, c is 0.1 to 10, d is 0.1 to 15, e is 0.001 to 10, and f is a number determined according to the oxidation state of each element, when a = 12.

In addition, the inorganic fiber may be one or more fibers selected from the group consisting of glass fiber, silica fiber, alumina fiber, and silica-alumina fiber. The inorganic fiber may have a number average length of 2 mm or less and a number average diameter of 2 to 40 $\mu$m. Further, the content of the inorganic fiber may be 2 to 15 parts by weight per 100 parts by weight of the active ingredient.

In addition, according to the present invention,

a preparation method of acrolein and acrylic acid, including the step of carrying out a catalytic vapor-phase oxidation reaction of one or more raw materials selected from the group consisting of propylene, isobutylene, and tert-butanol, and molecular oxygen, in the presence of the ring catalyst, is provided.

The catalytic vapor-phase oxidation reaction may be carried out in a multi-tubular fixed bed reactor in which the ring catalyst is packed.

In the preparation method, the ring catalyst may satisfy said Relational Equation 2. Furthermore, the ring catalyst may be packed in the reactor to form at least two layers having different $L_c/D_e$, and said $L_c/D_e$ may decrease from the raw material inlet side to the product outlet side of the reactor.

## [ADVANTAGEOUS EFFECTS]

The catalyst for preparing acrolein and acrylic acid according to the present invention can be uniformly packed in a reactor and the collapse of the catalyst can be minimized because it has excellent mechanical properties, and it can lower the initial pressure of the reaction by securing stable pore spaces and can be stably used for a long period of time. In addition, the preparation method of acrolein and acrylic acid according to the present invention can provide more improved production efficiency by using the catalyst.

## [BRIEF DESCRIPTION OF THE DRAWING]

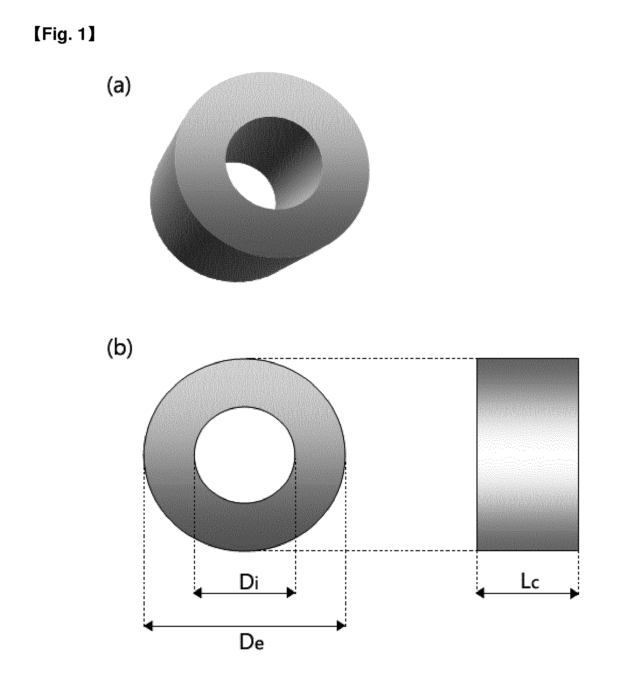

Fig. 1 shows (a) the perspective view and (b) the sectional view and side view of the catalyst according to one embodiment of the present invention, which are enlarged views.

<Explanation of Marks>

$D_i$: internal diameter of ring catalyst
$D_e$: external diameter of ring catalyst
$L_c$: longitudinal length of ring catalyst

## [DETAILED DESCRIPTION OF THE EMBODIMENTS]

Hereinafter, the catalyst for preparing acrolein and acrylic acid and the use of the same according to the embodiments of the present invention are explained by referring to the annexed drawing.

Prior to this, technical terms used in in the present specification are only for mentioning specific embodiments and they are not intended to restrict the present invention. The singular expressions used here may include the plural expressions unless they are differently expressed contextually. The meaning of the term "include" or "comprise" used in the specification embodies specific characteristics, areas, essence, steps, actions, elements, or components, and does not exclude existence or addition of other specific characteristics, areas, essence, steps, actions, elements, or components.

In the process of researching the catalyst for preparing acrolein and acrylic acid, the present inventors recognized that prior ring catalysts were easily broken by a shock in the process of preparation, storage, or packing in a reaction tube. Particularly, it was recognized that the broken fragments or dust of the catalyst raised the pressure in the reaction tube and made the pressure of each reaction tube irregular, in the case of multi-tubular reactor, and became a factor of decreasing the production efficiency.

Therefore, the present inventors repeated studies for resolving the problem, and recognized that the mechanical properties of the catalyst can be apparently improved by adding the inorganic fiber in company with the catalytic active ingredient in the process of preparing the ring catalyst and particularly by adjusting the ratio of the wall thickness of the ring catalyst and the number average length of the inorganic fiber to a specific range.

The present inventors also recognized that the decrease in effective dose of the catalytic active ingredient by using the ring catalyst can be minimized and more improved productivity can be secured, when the catalyst is applied to the preparation process of acrolein and acrylic acid.

According to one embodiment of the present invention,

a ring catalyst for preparing acrolein and acrylic acid,

including a mixture of an catalytic active ingredient including at least molybdenum (Mo) and bismuth (Bi), and an inorganic fiber, and

having a ring shape, and

satisfying the following Relational Equation 1,

is provided.

[Relational Equation 1]

$$0.1 \leq [2L_f/(D_e-D_i)] < 0.2$$

In said Relational Equation 1, $L_f$ is the number average length of the inorganic fiber, $D_e$ is the external diameter of the ring catalyst, and $D_i$ is the internal diameter of the ring catalyst.

The catalyst for preparing acrolein and acrylic acid according to the present invention includes the catalytic active ingredient and the inorganic fiber, and has a ring shape as illustrated in Fig.1 (a).

The catalyst may include a common component applied to the catalyst for preparing acrolein and acrylic acid as the active ingredient, and it is preferable for securing the catalytic activity to include at least molybdenum (Mo) and bismuth (Bi). More preferably, the catalytic active ingredient may be represented by the following Chemical Formula 1.

[Chemical Formula 1]        $MO_a Bi_b A_c B_d C_e Of$

In Chemical Formula 1,

Mo is molybdenum; Bi is bismuth; A is one or more elements selected from the group consisting of Fe, Zn, Mn, Nb, and Te; B is one or more elements selected from the group consisting of Co, Rh, and Ni; C is one or more elements selected from the group consisting of Na, K, Li, Cs, Ta, Ca, Rb, and Mg; O is oxygen; and

a, b, c, d, e, and f are atomic ratios of each element, wherein b is 0.1 to 10, c is 0.1 to 10, d is 0.1 to 15, e is 0.001 to 10, and f is a number determined according to the oxidation state of each element, when a = 12.

The catalytic active ingredient represented by Chemical Formula 1 can show excellent catalytic activity in the preparation of acrolein and acrylic acid, and thus makes it possible to provide more improved reaction activity.

Meanwhile, the catalyst according to the present invention includes the inorganic fiber uniformly mixed with the catalytic active ingredient. The catalyst according to the present invention can not only show improved mechanical properties but can also dissipate heat generated during the reaction, and makes it possible to suppress the consecutive reactions and the deterioration of the catalyst by heat.

The material of the inorganic fiber is not limited particularly, and common fibers in the art to which the present invention pertains can be used. For example, in order to sufficiently realize said effects without a negative influence on the catalytic activity, it is preferable that the inorganic fiber is one or more fibers selected from the group consisting of glass fiber, silica fiber, alumina fiber, and silica-alumina fiber.

Further, the content of the inorganic fiber included in the catalyst according to the present invention may be determined in the range in which said effect according to adding the inorganic fiber can be sufficiently exhibited and the catalytic activity does not decrease. For a non-restrictive example, the content of the inorganic fiber may be 2 to 15 parts by weight, preferably 2 to 10 parts by weight, per 100 parts by weight of the catalytic active ingredient.

Particularly, according to the present invention, the mechanical properties of the ring catalyst may markedly differ according to the number average length of the inorganic fiber and the wall thickness of the ring catalyst. Preferably, the catalyst according to the present invention may satisfy the following Relational Equation 1.

## [Relational Equation 1]

$$0.1 \leq [2L_f/(D_e-D_i)] < 0.2$$

In said Relational Equation 1, $L_f$ is the number average length of the inorganic fiber, $D_e$ is the external diameter of the ring catalyst, and $D_i$ is the internal diameter of the ring catalyst.

That is, even when the ring catalyst includes the inorganic fiber, if the length of the inorganic fiber is too long or too short in comparison to the wall thickness of the ring catalyst, the effect of adding the inorganic fiber decreases and it may rather act as a factor decreasing activity of the catalyst.

In this viewpoint, as illustrated in Fig. 1 (b), the catalyst according to the present invention is designed so that the number average length ($L_f$) of the inorganic fiber included in the catalyst is differently selected according to the wall thickness ($[(D_e-D_i)/2]$) of the catalyst, and particularly, the ratio $[2L_f/(D_e-D_i)]$ thereof satisfies said Relational Equation 1. Preferably, the ratio $[2L_f/(D_e-D_i)]$ may be 0.1 to 0.19, 0.12 to 0.19, 0.12 to 0.18, 0.12 to 0.16, or 0.14 to 0.16. Accordingly, the catalyst according to the present invention may include the inorganic fiber which makes the catalyst exhibit the optimal mechanical property improvement effect according to the wall thickness of the catalyst.

According to the present invention, the catalytic active ingredient may be supported somewhat on inert supporting materials even when $[2L_f/(D_e-D_i)]$ in said Relational Equation 1 is less than 0.1, or 0.2 or more. However, when $[2L_f/(D_e-D_i)]$ is less than 0.1, it is not preferable because the overall mechanical properties of the catalyst may decrease, for example, the attrition rate of the catalyst increases and the shatter strength decreases. Further, when $[2L_f/(D_e-D_i)]$ is 0.2 or more, it is not preferable because the mechanical properties of the catalyst may decrease somewhat and the activity of the catalyst may decrease.

In the present invention, the wall thickness ($[(D_e-D_i)/2]$) of the catalyst may be 0.5 mm or more, preferably 0.5 to 5.0 mm, and more preferably 1.0 to 4.0 mm, for securing basic structural stability.

The number average length ($L_f$) of the inorganic fiber may be 2 mm or less (for a non-restrictive example, 0.05 to 2 mm, 0.1 to 1.5 mm, or 0.1 to 1 mm) to be uniformly mixed with the catalytic active ingredient, and it may be determined according to the wall thickness of the catalyst in the range satisfying said Relational Equation 1.

The number average diameter of the inorganic fiber may be 2 $\mu$m or more, preferably 2 to 40 $\mu$m, and more preferably 4 to 20 $\mu$m, for exhibiting the effect by adding the inorganic fiber.

Meanwhile, the catalyst according to the present invention has the shape satisfying the following Relational Equation 2.

## [Relational Equation 2]

$$0.2 \leq L_c/D_e \leq 1.5$$

In said Relational Equation 2, $L_c$ is the longitudinal length of the ring catalyst, and $D_e$ is the external diameter of the ring catalyst.

That is, as shown in Fig. 1 (b), the catalyst according to the present invention has a ring shape (that is, a hollow cylinder shape), and here, the longitudinal length ($L_c$) of the ring catalyst may be determined in a range in which the unique properties of the ring catalyst (for example, ability to secure a wider pore space and to dissipate reaction heat) can be exhibited, and preferably it may be determined in the range satisfying said Relational Equation 2 in the relationship with the external diameter ($D_e$) of the ring catalyst.

The ring catalyst of the present invention may have at least two shapes having different $L_c/D_e$. That is, the ring catalyst is used in the reaction in the state of being packed in a catalytic vapor-phase oxidation reactor for preparing acrolein and acrylic acid, and it is possible to form catalyst layers having different catalyst packing densities by using at least two catalysts having different $L_c/D_e$.

For a non-restrictive example, the ring catalyst may include 2 kinds or more of catalysts of the first type having $L_c/D_e$ of 0.2 to 1, 0.2 to 0.9, 0.2 to 0.7, 0.3 to 0.7, or 0.3 to 0.5; and the second type having $L_c/D_e$ of 0.6 to 1.5, 0.6 to 1.3, 0.7 to 1.3, 0.8 to 1.2, 0.9 to 1.2, or 1 to 1.2.

In this way, the packing density of the catalyst layers in the reactor may be regulated by controlling the shape of the ring catalyst as necessary. Further, the preparation efficiency of acrolein and acrylic acid can be improved through the packing density control of the ring catalyst. The packing method of the ring catalyst will be explained in more detail in the description below related to the preparation method of acrolein and acrylic acid.

Meanwhile, the catalyst according to the present invention may be prepared by the method of forming a powdery mixture including the catalytic active ingredient and the inorganic fiber into a ring shape as illustrated in Fig. 1(a), and firing the same. At this time, it is advantageous in the aspect of sufficient exhibition of the catalytic activity to carry out the firing step under an oxygen atmosphere at 300 to 700 °C for 2 to 7 h.

Meanwhile, according to another embodiment of the present invention, a preparation method of acrolein and acrylic acid, including the step of carrying out a catalytic vapor-phase oxidation reaction of one or more raw materials selected from the group consisting of propylene, isobutylene, and tert-butanol, and molecular oxygen, in the presence of said catalyst, is provided.

That is, the ring catalyst disclosed above can show excellent activity when it is applied to the preparation of acrolein and acrylic acid.

The raw material of the preparation method may be a common compound in the art to which the present invention pertains, for example, one or more compounds selected from the group consisting of propylene, isobutylene, and tert-butanol. The raw material can be converted into acrolein and acrylic acid through a catalytic vapor-phase oxidation reaction with molecular oxygen.

At this time, the catalytic vapor-phase oxidation reaction may be carried out in a multi-tubular fixed bed reactor packed with the ring catalyst. The multi-tubular fixed bed reactor may be equipped with a shell and tube type of heat exchanger, and except for this, a reactor having a common structure in the art to which the present invention pertains may be used without particular limitation.

Particularly, since the ring catalyst satisfying said Relational Equation 1 and having excellent mechanical properties is applied to the preparation method of acrolein and acrylic acid according to the present invention, uniform packing of the catalyst is possible and collapse of the catalyst can be minimized. Further, since the pore space can be stably secured and the initial pressure of the reaction can be maximally lowered by applying said catalyst to the preparation method, it becomes possible to stably operate the process for a long period of time.

Furthermore, the ring catalyst for the preparation method of acrolein and acrylic acid satisfies said Relational Equation 2. Particularly, the ring catalyst may be packed in the reactor (each reaction tube in the case of the multi-tubular fixed bed reactor) to form at least two layers having different $L_c/D_e$, and, the ring catalyst may be packed so that said $L_c/D_e$ decreases from the raw material inlet to the product outlet of the reactor.

In this regard, common ring catalysts can exhibit excellent heat dissipation ability because they can secure a wider pore space in the reactor than pellet catalysts (that is, packing density of the same is lower), but it has a disadvantage in that the content of the catalytic active ingredient in the reactor is relatively low. In this way, when a ring catalyst is used, the content of the effective ingredient (that is, the catalytic active ingredient) decreases, and thus there is a disadvantage in that it is difficult to operate the process for a long time and the production efficiency decreases because the lifespan of the catalyst shortens.

However, according to the present invention, said problems can be resolved by setting up the packing density of the ring catalyst packed in the reactor differently through the shape control of the catalyst. That is, in the present invention, the ring catalyst may be packed in the reactor to form at least two layers having different $L_c/D_e$, and particularly, it may be packed so that said $L_c/D_e$ decreases from the raw material inlet to the product outlet of the reactor.

As a non-restrictive example, the first layer (the catalyst layer of the raw material inlet side) in which the ring catalyst of $L_c/D_e = 1.0$ is packed and the second layer (the catalyst layer of the product outlet side) in which the ring catalyst of $L_c/D_e = 0.4$ is packed may be formed in the reactor. By this, relatively low packing density is provided to the catalyst layer of the raw material inlet side, and relatively high packing density is provided to the later section.

Accordingly, the preparation method of the present invention can secure the reaction activity and the heat dissipation effect at the same time by providing wider pore space to the raw material inlet side where the reaction occurs actively, and the content of the effective ingredient (catalytic active ingredient) packed in the whole reactor can be increased by providing higher packing density to the section after the catalyst layer of the raw material inlet side. Therefore, the present method can compensate for life shortening of the catalyst according to the decrease of the packed effective ingredient, and stable operation of the process is possible for a long period of time.

The $L_c/D_e$ control range of the ring catalyst according to the section of the reactor may be determined in a range in which said effects can be sufficiently exhibited and sufficient catalytic activity can be secured.

As a non-restrictive example, the ring catalyst may include 2 kinds or more of catalysts of the first type having $L_c/D_e$ of 0.2 to 1, 0.2 to 0.9, 0.2 to 0.7, 0.3 to 0.7, or 0.3 to 0.5; and the second type having $L_c/D_e$ of 0.6 to 1.5, 0.6 to 1.3, 0.7 to 1.3, 0.8 to 1.2, or 1 to 1.2.

Furthermore, when two catalyst layers having different $L_c/D_e$ are packed in the reactor, it is possible to form the catalyst layer including the first type of catalyst of which $L_c/D_e$ is 0.6 to 1.5 at the raw material inlet side, and the catalyst layer including the second type of catalyst of which $L_c/D_e$ is 0.2 to 1 at the later section. However, the first type of catalyst and the second type of catalyst have different $L_c/D_e$ from each other. At this time, the length of each catalyst layer may be determined by considering the reaction efficiency and so on, and it is not particularly limited.

Meanwhile, the mole ratio of the raw materials and oxygen put in the reactor may be 1:0.5 to 1:3, and the reaction may be carried out at 200 to 450 °C under a pressure of 0.1 to 10 atm.

Hereinafter, preferable examples and comparative examples are presented for better understanding of the present invention. However, the following examples are only for illustrating the present invention and the present invention is not limited to or by them.

Preparation Example (preparation of catalytic active ingredient)

A 1st solution was prepared by dissolving about 1000 g of ammonium molybdate in about 2500 ml of distilled water in a 5 L glass reactor equipped with a stirrer while heating the same to about 90 °C.

Separately, a 2nd solution was prepared by dissolving about 84 g of nitric acid in about 500 ml of distilled water while adding about 503.73 g of bismuth nitrate, about 267 g of iron nitrate, about 755.54 g of cobalt nitrate, and about 19.09 g of potassium nitrate thereto and mixing the same.

A suspension was prepared by mixing the 1st solution and the 2nd solution while maintaining the temperature at about 40 °C. After drying the prepared suspension in an electric oven at about 130 °C for about 24 h, a powdery material was obtained by pulverizing the dried material to a diameter of about 130 $\mu$m or less while stirring for about 2 h.

Example 1

A mixture including 5 parts by weight of silica-alumina fiber (number average diameter: about 7 $\mu$m, number average length ($L_f$): about 225 $\mu$m) per 100 parts by weight of the catalytic active ingredient according to Preparation Example was stirred for about 30 min. The mixture was extruded into a ring shape having an about 5 mm external diameter ($D_e$), an about 2 mm internal diameter ($D_i$), and an about 5.5 mm longitudinal length ($L_c$) ($[2L_f/(D_e-D_i)]$ = about 0.15, $L_c/D_e$ = about 1.1). The catalyst was obtained by firing the extruded article under an oxygen atmosphere at about 500 °C for about 5 h. The composition ratio of the elements except oxygen in the catalytic active ingredient was recognized as $Mo_{12}Bi_{2.2}Fe_{1.4}Co_{5.5}K_{0.4}$.

Example 2

A mixture including 5 parts by weight of silica-alumina fiber (number average diameter: about 7 $\mu$m, number average length ($L_f$): about 225 $\mu$m) per 100 parts by weight of the catalytic active ingredient according to Preparation Example was stirred for about 30 min. The mixture was extruded into a ring shape having an about 5 mm external diameter ($D_e$), an about 2 mm internal diameter ($D_i$), and an about 2.5 mm longitudinal length ($L_c$) ($[2L_f/(D_e-D_i)]$ = about 0.15, $L_c/D_e$ = about 0.5). The catalyst was obtained by firing the extruded article under an oxygen atmosphere at about 500 °C for about 5 h.

Example 3

A mixture including 5 parts by weight of silica-alumina fiber (number average diameter: about 7 $\mu$m, number average length ($L_f$): about 150 $\mu$m) per 100 parts by weight of the catalytic active ingredient according to Preparation Example was stirred for about 30 min. The mixture was extruded into a ring shape having an about 5 mm external diameter ($D_e$), an about 2 mm internal diameter ($D_i$), and an about 5.5 mm longitudinal length ($L_c$) ($[2L_f/(D_e-D_i)]$ = about 0.1, $L_c/D_e$ = about 1.1). The catalyst was obtained by firing the extruded article under an oxygen atmosphere at about 500 °C for about 5 h.

Example 4

A mixture including 5 parts by weight of silica-alumina fiber (number average diameter: about 7 $\mu$m, number average length ($L_f$): about 180 $\mu$m) per 100 parts by weight of the catalytic active ingredient according to Preparation Example was stirred for about 30 min. The mixture was extruded into a ring shape having an about 5 mm external diameter ($D_e$), an about 2 mm internal diameter ($D_i$), and an about 5.5 mm longitudinal length ($L_c$) ($[2L_f/(D_e-D_i)]$ = about 0.12, $L_c/D_e$ = about 1.1). The catalyst was obtained by firing the extruded article under an oxygen atmosphere of about 500 °C for about 5 h.

Example 5

A mixture including 5 parts by weight of silica-alumina fiber (number average diameter: about 7 $\mu$m, number average length ($L_f$): about 285 $\mu$m) per 100 parts by weight of the catalytic active ingredient according to Preparation Example was stirred for about 30 min. The mixture was extruded into a ring shape having an about 5 mm external diameter ($D_e$), an about 2 mm internal diameter ($D_i$), and an about 5.5 mm longitudinal length ($L_c$) ($[2L_f/(D_e-D_i)]$ = about 0.19, $L_c/D_e$ = about 1.1). The catalyst was obtained by firing the extruded article under an oxygen atmosphere at about 500 °C for about 5 h.

Comparative Example 1

The catalyst ([$2L_f/(D_e-D_i)$] = about 0.075, $L_c/D_e$ = about 1.1) was obtained by the same method as in Example 1, except that silica-alumina fiber having a number average length ($L_f$) of about 112.5 $\mu$m was used.

Comparative Example 2

The catalyst ([$2L_f/(D_e-D_i)$] = about 0.25, $L_c/D_e$ = about 1.1) was obtained by the same method as in Example 1, except that a silica-alumina fiber having number average length ($L_f$) of about 375 $\mu$m was used.

Comparative Example 3

The catalyst ([$2L_f/(D_e-D_i)$] = about 0.35, $L_c/D_e$ = about 1.1) was obtained by the same method as in Example 1, except that a silica-alumina fiber having number average length ($L_f$) of about 525 $\mu$m was used.

Comparative Example 4

The catalyst was obtained by the same method as in Example 1, except that a silica-alumina fiber was not added thereto.

Comparative Example 5

The powder obtained by drying and pulverizing the suspension in Preparation Example was formed into a cylinder form having an external diameter of 5 mm and a length of 5.5 mm, and the cylinder type of catalyst was obtained by firing the same under an oxygen atmosphere at about 500 °C for about 5 h.

Experimental Example 1 (measurements on mechanical properties of the catalyst)

Strength, attrition rate, and shatter strength were measured for each catalyst according to the examples and comparative examples by the following methods, and the results are listed in the following Table 1.

1) Impact Strength (kgf/cm$^2$): measured by using grain crushing strength tester (model name: GCS Tester (ASTM D-4179 & D-6175), manufacturer: VINCI Technologies).
2) Attrition Rate (%): measured with a 30 rpm condition for 30 min by using an attrition tester (model name: Rotating Drum Attrition Tester (ASTM D-4058-96), manufacturer: VINCI Technologies).
3) Shatter Strength (%): after covering the bottom of a 6000 mm diameter reaction tube with a 2 mm mesh, 100 g of the catalyst was dropped into the reaction tube, and then sieved by using a 3.5 mm mesh. The weight of the catalyst left on the 3.5 mm mesh was measured and the shatter strength was calculated according to the following equation.

$$\text{Shatter Strength} = [(\text{Weight of the catalyst left on the mesh})/(\text{Weight of the dropped catalyst})*100]$$

[Table 1]

|  | Shape | $2L_f/(D_e-D_i)$ | $L_c/D_e$ | Impact Strength (kgf/cn$^2$) | Attrition Rate (%) | Shatter Strength (%) |
|---|---|---|---|---|---|---|
| Example 1 | Ring | 0.15 | 1.1 | 2.4 | 6.1 | 96.9 |
| Example 2 | Ring | 0.15 | 0.5 | 1.1 | 7.8 | 98.1 |
| Example 3 | Ring | 0.1 | 1.1 | 2.2 | 6.1 | 96.4 |
| Example 4 | Ring | 0.12 | 1.1 | 2.2 | 6.3 | 96.8 |
| Example 5 | Ring | 0.19 | 1.1 | 2.5 | 6.0 | 96.8 |

(continued)

|  | Shape | $2L_f/(D_e-D_i)$ | $L_c/D_e$ | Impact Strength (kgf/cn$^2$) | Attrition Rate (%) | Shatter Strength (%) |
|---|---|---|---|---|---|---|
| Comparative Example 1 | Ring | 0.075 | 1.1 | 2.3 | 6.4 | 95.5 |
| Comparative Example 2 | Ring | 0.25 | 1.1 | 2.1 | 6.3 | 96.2 |
| Comparative Example 3 | Ring | 0.35 | 1.1 | 2.3 | 6.4 | 95.4 |
| Comparative Example 4 | Ring | - | 1.1 | 1.8 | 18.9 | 57.7 |
| Comparative Example 5 | Cylinder | - | 1.1 | 3.2 | 12 | 85.1 |

Experimental Example 2 (measurement on activity of the catalyst)

(1) Acrolein and acrylic acid were prepared by a catalytic vapor-phase oxidation reaction of propylene, a raw material, in the presence of each catalyst according to the examples and comparative examples. The reaction was carried out in a multi-tubular fixed bed reactor equipped with a shell and tube type of heat exchanger (internal diameter of tube: 1 inch (25.4 mm), diameter of shell: 350 mm, length of catalyst-packing section: 3000 mm), and the reaction was carried out at a reaction temperature of about 305 °C while streaming the raw material mixture gas (propylene: 8 volume%, oxygen: 14 volume%, water vapor: 18 volume%, inert gas: 60 volume%) with a space velocity of 1500 h$^{-1}$. (2) Acrolein and acrylic acid were prepared by a catalytic vapor-phase oxidation reaction of propylene, a raw material, in the presence of two catalysts according to Examples 1 and 2. The reaction was carried out in a multi-tubular fixed bed reactor equipped with a shell and tube type of heat exchanger (internal diameter of tube: 1 inch (25.4 mm), diameter of shell: 350 mm, length of catalyst-packing section - catalyst of Example 1: 2000 mm at the raw material mixture gas inlet side, the catalyst of Example 2: 1000 mm at the later section), and the reaction was carried out at a reaction temperature of about 305 °C while streaming the raw material mixture gas (propylene: 8 volume%, oxygen: 14 volume%, water vapor: 18 volume%, inert gas: 60 volume%) with a space velocity of 1500 h$^{-1}$.

The conversion rate of propylene, the selectivity of acrolein, and the yield were calculated by the following equations and the results are listed in the following Table 2.

1) Conversion rate of propylene (%) = [(mole of propylene reacted)/(mole of propylene provided)]*100

2) Selectivity of acrolein (%) = [(mole of acrolein produced)/(mole of propylene reacted)]*100

3) Yield (%) = [(mole of acrolein and acrylic acid produced)/(mole of propylene provided)]*100

[Table 2]

| Catalyst | Conversion Rate of Propylene (%) | Selectivity of Acrolein (%) | Yield (%) |
|---|---|---|---|
| Example 1 | 98.8 | 93.6 | 92.5 |
| Example 2 | 99.5 | 94.4 | 93.9 |
| Example 3 | 98.6 | 93.6 | 92.3 |

(continued)

| Catalyst | Conversion Rate of Propylene (%) | Selectivity of Acrolein (%) | Yield (%) |
|---|---|---|---|
| Example 4 | 98.8 | 93.5 | 92.4 |
| Example 5 | 98.5 | 93.8 | 92.4 |
| Examples 1 & 2 | 99.2 | 94.1 | 93.3 |
| Comparative Example 1 | 98.4 | 93.6 | 92.1 |
| Comparative Example 2 | 98.4 | 93.6 | 92.1 |
| Comparative Example 3 | 98.5 | 93.2 | 91.8 |
| Comparative Example 4 | 98.6 | 93.1 | 91.8 |
| Comparative Example 5 | 98.3 | 92.0 | 90.4 |

As shown in Tables 1 and 2, it is recognized that the catalysts according to the examples are superior to the catalysts according to the comparative examples not only in the catalytic activity but also in the mechanical properties such as impact strength, attrition rate, shatter strength, and so on.

Further, it is recognized that the case of packing the catalyst of Example 1 at the raw material mixture gas inlet and the catalyst of Example 2 at the later section is superior to the case of using the catalyst of Example 2 solely for mechanical stability of the catalyst layer and lifespan of the catalyst while securing equivalent catalytic activity.

**Claims**

1. A ring catalyst for preparing acrolein and acrylic acid,
   including a mixture of an catalytic active ingredient including at least molybdenum (Mo) and bismuth (Bi), and an inorganic fiber, and
   having a ring shape, and
   satisfying the following Relational Equation 1:

[Relational Equation 1]

$$0.1 \leq [2L_f/(D_e - D_i)] < 0.2$$

wherein, in said Relational Equation 1, $L_f$ is the number average length of the inorganic fiber, $D_e$ is the external diameter of the ring catalyst, and $D_i$ is the internal diameter of the ring catalyst.

2. The ring catalyst according to Claim 1, satisfying the following Relational Equation 2:

[Relational Equation 2]

$$0.2 \leq L_c/D_e \leq 1.5$$

wherein, in said Relational Equation 2, $L_c$ is the longitudinal length of the ring catalyst, and $D_e$ is the external diameter of the ring catalyst.

3. The ring catalyst according to Claim 2, wherein the ring catalyst has at least two shapes having different $L_c/D_e$.

4. The ring catalyst according to Claim 3, wherein the ring catalyst includes 2 kinds or more of catalysts of the first type having $L_c/D_e$ of 0.2 to 1 and the second type having $L_c/D_e$ of 0.6 to 1.5.

5. The ring catalyst according to Claim 1, wherein the catalytic active ingredient is represented by the following Chemical Formula 1:

[Chemical Formula 1]

$$Mo_a\ Bi_b\ A_c\ B_d\ C_e\ O_f$$

wherein, in Chemical Formula 1,
Mo is molybdenum; Bi is bismuth; A is one or more elements selected from the group consisting of Fe, Zn, Mn, Nb, and Te; B is one or more elements selected from the group consisting of Co, Rh, and Ni; C is one or more elements selected from the group consisting of Na, K, Li, Cs, Ta, Ca, Rb, and Mg; O is oxygen; and
a, b, c, d, e, and f are atomic ratios of each element, wherein b is 0.1 to 10, c is 0.1 to 10, d is 0.1 to 15, e is 0.001 to 10, and f is a number determined according to the oxidation state of each element, when a = 12.

6. The ring catalyst according to Claim 1, wherein the inorganic fiber is one or more fibers selected from the group consisting of glass fiber, silica fiber, alumina fiber, and silica-alumina fiber.

7. The ring catalyst according to Claim 1, wherein the inorganic fiber a the number average length of 2 mm or less and a number average diameter of 2 to 40 $\mu$m.

8. The ring catalyst according to Claim 1, wherein the content of the inorganic fiber is 2 to 15 parts by weight per 100 parts by weight of the active ingredient.

9. A preparation method of acrolein and acrylic acid, including the step of carrying out a catalytic vapor-phase oxidation reaction of one or more raw materials selected from the group consisting of propylene, isobutylene, and tert-butanol, and molecular oxygen, in the presence of the ring catalyst according to Claim 1.

10. The preparation method according to Claim 9, wherein the catalytic vapor-phase oxidation reaction is carried out in a multi-tubular fixed bed reactor in which the ring catalyst is packed.

11. The preparation method according to Claim 9, wherein the ring catalyst satisfies the following Relational Equation 2:

[Relational Equation 2]

$$0.2 \leq L_c/D_e \leq 1.5$$

wherein, in said Relational Equation 2, $L_c$ is the longitudinal length of the ring catalyst, and $D_e$ is the external diameter of the ring catalyst.

12. The preparation method according to Claim 10, wherein the ring catalyst is packed in the reactor to form at least two layers having different $L_c/D_e$, and said $L_c/D_e$ decreases from the raw material inlet side to the product outlet side of the reactor.

【Fig. 1】

(a)

(b)

| INTERNATIONAL SEARCH REPORT | International application No. |
|---|---|
| | **PCT/KR2014/004670** |

| A. CLASSIFICATION OF SUBJECT MATTER |
|---|
| *B01J 23/31(2006.01)i, B01J 35/02(2006.01)i, B01J 37/08(2006.01)i, C07C 57/04(2006.01)i, C07C 47/22(2006.01)i* |
| According to International Patent Classification (IPC) or to both national classification and IPC |

| B. FIELDS SEARCHED |
|---|
| Minimum documentation searched (classification system followed by classification symbols) |
| B01J 23/31; B01J 23/88; B01J 37/00; C07C 45/35; B01J 23/16; B01J 35/02; B01J 37/08; C07C 57/04; C07C 47/22 |

| Documentation searched other than minimum documentation to the extent that such documents are included in the fields searched |
|---|
| Korean Utility models and applications for Utility models: IPC as above |
| Japanese Utility models and applications for Utility models: IPC as above |

| Electronic data base consulted during the international search (name of data base and, where practicable, search terms used) |
|---|
| eKOMPASS (KIPO internal) & Keywords: acrolein, acrylic acid, catalyst, ring, molybdenum, bismuth, inorganic fiber, length, external diameter, inside diameter, propylene, isobutylene, tertiary butanol, oxidation |

| C. DOCUMENTS CONSIDERED TO BE RELEVANT |
|---|

| Category* | Citation of document, with indication, where appropriate, of the relevant passages | Relevant to claim No. |
|---|---|---|
| X | KR 10-2002-0075281 A (NIPPON CATALYTIC CHEM. IND.) 04 October 2002 See abstract; pages 1-6; claims 1-6; table 1. | 1-12 |
| A | JP 07-185349 A (MITSUBISHI RAYON CO., LTD.) 25 July 1995 See abstract; paragraphs [0005]-[0007], [0010], [0013], [0015], [0018], [0026]. | 1-12 |
| A | JP 2005-211796 A (MITSUBISHI CHEM. CORP.) 11 August 2005 See abstract; paragraphs [0016]-[0019], [0024]. | 1-12 |
| A | KR 10-2009-0079835 A (LG CHEM. LTD.) 22 July 2009 See abstract; paragraphs [25]-[35], [46], [68]; claims 1-3, 10, 14. | 1-12 |
| A | KR 10-2003-0070105 A (BASF AG.) 27 August 2003 See abstract; pages 2, 4-6; claims 1, 4-6, 8, 11, 15; figures 4A, 4B. | 1-12 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29 AUGUST 2014 (29.08.2014) | **01 SEPTEMBER 2014 (01.09.2014)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

**INTERNATIONAL SEARCH REPORT**
Information on patent family members

International application No.

**PCT/KR2014/004670**

| Patent document cited in search report | Publication date | Patent family member | Publication date |
|---|---|---|---|
| KR 10-2002-0075281 A | 04/10/2002 | BR 0200854 A | 25/03/2003 |
| | | CN 1232348 C | 21/12/2005 |
| | | CN 1386579 A | 25/12/2002 |
| | | EP 1243331 A1 | 25/09/2002 |
| | | JP 03892244 B2 | 14/03/2007 |
| | | JP 2002-273229 A | 24/09/2002 |
| | | KR 10-0709055 B1 | 18/04/2007 |
| | | SG 103339 A1 | 29/04/2004 |
| | | TW I299006 B | 21/07/2008 |
| | | US 2002-0198103 A1 | 26/12/2002 |
| | | US 2004-0199008 A1 | 07/10/2004 |
| | | US 6784134 B2 | 31/08/2004 |
| | | US 6878847 B2 | 12/04/2005 |
| | | ZA 200202265 A | 11/10/2002 |
| JP 07-185349 A | 25/07/1995 | JP 03313863 B2 | 12/08/2002 |
| JP 2005-211796 A | 11/08/2005 | CN 1697697 A | 16/11/2005 |
| | | WO 2005-072869 A1 | 11/08/2005 |
| KR 10-2009-0079835 A | 22/07/2009 | CN 101918126 A | 15/12/2010 |
| | | KR 10-1043400 B1 | 22/06/2011 |
| | | US 2010-0298601 A1 | 25/11/2010 |
| | | WO 2009-091212 A2 | 23/07/2009 |
| | | WO 2009-091212 A3 | 29/10/2009 |
| KR 10-2003-0070105 A | 27/08/2003 | AU 2002-246034 A8 | 19/08/2002 |
| | | EP 1353892 A2 | 22/10/2003 |
| | | JP 2004-517953 A | 17/06/2004 |
| | | US 2004-0054222 A1 | 18/03/2004 |
| | | US 2007-0032680 A1 | 08/02/2007 |
| | | US 7129195 B2 | 31/10/2006 |
| | | WO 02-062737 A2 | 15/08/2002 |
| | | WO 02-062737 A3 | 07/11/2002 |

Form PCT/ISA/210 (patent family annex) (July 2009)